# EUROPEAN PATENT SPECIFICATION

(11) **EP 0 534 955 B1**
(45) Date of publication and mention of the grant of the patent: **28.05.1997**
(21) Application number: 90911243.5
(22) Date of filing: 12.07.1990
(51) Int. Cl.: C12P 23/00, C12N 1/20, C12N 9/14, A01N 63/00, A23L 1/28

(54) **Process for the production of a zeaxanthin-containing composition using a microorganism of the species Flavobacterium multivorum**
Verfahren zur Herstellung einer Zeaxanthin enthaltenden Zusammensetzung mittels eines Mikroorganismus der Spezies Flavobacterium multivorum
Procédé pour la production d'une composition contenant de la zéaxanthine au moyen d'un microoganisme de l'espèce Flavobacterium multivorum

(30) Priority: 30.08.1989 US 400396
(43) Date of publication of application: 07.04.1993
(73) Proprietor: APPLIED FOOD BIOTECHNOLOGY, INC., Fenton, MO 63026 (US)
(72) Inventor: GIERHART, Dennis, L., High Ridge, MO 63049 (US)
(74) Representative: Nicholls, Kathryn Margaret
(86) International application number: PCT/US90/03970
(87) International publication number: WO 91/03571

(56) References cited:
- CH-A- 600 791
- US-A- 2 515 135
- US-A- 2 974 044
- US-A- 3 891 504
- US-A- 3 920 834
- US-A- 4 001 084
- US-A- 4 026 949
- US-A- 4 039 384
- US-A- 4 642 131
- US-A- 4 713 340
- METHODS IN ENZYMOLOGY, vol. 67, part F, "Vitamins and Coenzymes", 1980; G. BRITTON et al., pp. 264-270
- American Type Culture Collection Catalogue of Bacteria, Phages and rDNA Vectors, Sixteenth Edition, 1985, page 209
- The National Collection of Industrial Bacteria Catalogue of Strains, Third Edition, 1975, pages 98 and 110
- ARCHIVES OF MICROBIOLOGY, vol. 113, issued 1977, BRITTON et al., pp. 33-37
- AQUACULTURE, vol. 20, issued 1980, Amsterdam (NL); JOHNSON et al.; pp. 123-134
- STANBURY et al.; "Principles of Fermentation Technology", 1984, Pergamon Press, pp. 77-82

## Description

The present invention involves the production of zeaxanthin using a microorganism of the species Flavobacterium multivorum, nutrient media for culturing and fermenting F. multivorum, compositions which include F. multivorum cell particles, and compositions which include zeaxanthin produced by a microorganism of this invention.

### BACKGROUND AND SUMMARY OF THE INVENTION

Zeaxanthin (3,3'-dihydroxy-β-carotene) is a carotenoid which imparts the yellow color to corn, egg yolks and the skin of poultry. It can be used as a feed additive, and as a colorant in the cosmetic and food industries.

The pure zeaxanthin and the pigment-containing cell-mass manufactured according to the process of this invention can be used for the coloring of foodstuffs, as well as for the coloring of cosmetic preparations. The pigment-containing cell-mass is particularly suitable for the coloring of legs, beaks, skin, fat, flesh and egg-yolk of poultry.

Zeaxanthin is synthesized biologically by very few bacterial species of the genus Flavobacterium. With rare exceptions, all Flavobacterium species may be conveniently placed into two categories: 1) strongly proteolytic (digestion of gelatin, casein, and coagulated serum), and 2) nonproteolytic. F. meningosepticum (biovar IIa) and F. indologenes (biovar IIb) are always proteolytic; other flavobacteria are not (IIc, IIe, IIh, IIi, and IIk-2). F. multivorum is one of three species now recognized in CDC's IIk-2 group (non-proteolytic).

For poultry producers, there has been a long history of problems with stability and biological availability, particularly with xanthophylls from marigolds and alfalfa, Much work goes on to measure and improve these properties. Most marigold products must be solvent-extracted and saponified, and may require the inclusion of antioxidants in the extraction process (Marusich and Bauerfeind, Carotenoids As Colorants and Vitamin A Precursors; ed., J.C. Bauerfeind, Academic Press, 1981). The feeding studies shown in Example 6 directly compared a known amount of pigment versus several processed extracts from marigolds; the data indicate that the composition of the present invention is biologically available and stable, pigments faster, and is 2-3 times more potent, on a pure pigment basis, than marigold xanthophylls. It is known that several species called Flavobacterium, under certain process conditions and/or using certain nutrient media, are capable of producing zeaxanthin (see the Relevant Literature section).

It is also known that improved yields of zeaxanthin may be obtained by culturing a microorganism designated Flavobacterium under conditions whereby the amounts of carbon and nitrogen present in the culture medium are maintained at a substantially constant ratio. The nutrient media of these processes require glucose and other nutrients, which are relatively costly and require a long culturing period.

On the other hand, the present invention provides a process for preparing zeaxanthin by culturing a zeaxanthin-producing microorganism of Flavobacterium multivorum, the first time that this species of Flavobacterium has been shown to produce pigment. Moreover, the nutrient medium in which the microorganism is cultured is a relatively low cost nutrient medium. Furthermore, the culturing period is much faster and more efficient than known methods.

Accordingly, an object of the present invention is to provide a process for the preparation of zeaxanthin using a strain of Flavobacterium multivorum, or a mutant or variant thereof. The process according to the present invention makes it possible to obtain increased amounts of zeaxanthin and increased cell yields of zeaxanthin containing cells in comparison to known microorganisms and processes.

It is another object to provide a process for producing increased yields of the biologically available zeaxanthin pigment.

It is another object to provide a process for producing zeaxanthin using microorganisms which are non-fastidious, grow rapidly, and are non-pathogenic. These microorganisms are also capable of using a nutrient medium containing many different carbon sources.

It is another object to provide an economical and commercially feasible nutrient medium which optimizes the amount of zeaxanthin produced, provides a high cell yield, provides a high yield of zeaxanthin, and decreases the fermentation period for producing zeaxanthin.

Another object is to provide a bacterial species that produces zeaxanthin.

Another object is to provide a microorganism heretofore not known to produce zeaxanthin.

These objects and others will become apparent in the following description of the invention and in the claims.

### RELEVANT LITERATURE

U.S. patent 3,891,504 discloses compositions and a method for producing zeaxanthin. The two Flavobacterium species are identified as ATCC Nos. 21588 and 21081. These strains were fermented in a nutrient medium, which included glucose, tryptone, and a yeast extract, and were subjected to a process that included temperature shifts and the use of pigment promoters (lactic acid and palmitic acid methylesters). These bacteria are listed as "Unidentified Bacteria" in the 1985 catalogue of the ATCC. As noted below (p22), ATCC 21588 and 21081 would not be classified in the Flavobacterium genus according to the taxonomic scheme described in the 1984 edition of Bergey's manual.

U.S. Patents 3,841,967; 3,951,742; and 3,951,743 disclose the use of Flavobacterium strains (identified as ATCC Nos. 21588, 21081 and 11947- from the same lineage as ATCC 21588 and 21081 -) in a process for producing zeaxanthin using a method similar to that for β carotene production. The process involves the growth of the microorganism in a batch-feed glucose nutrient medium, with the exception that the culture is continuously fed additional nutrients to maintain a constant carbon/nitrogen ratio. These patents also disclose a mutation procedure for isolating high pigment-producing bacterial strains. The process using this mutant produces high levels of zeaxanthin, but it is not economical.

U.S. patent 4,026,949 discloses the production of optically active intermediates used in the production of carotenoids, such as zeaxanthin; the bacteria used in this process is Flavobacterium dehydrogenans.

Arch Microbiol, 113, 33-37 (1977) describes the production of zeaxanthin from certain bacteria which were designated "Flavobacterium" by the authors. These include "Flavobacterium rhenanum". The 1975 catalogue of the NCIB indicates this is more properly classified as Erwinia.

### SPECIFIC DESCRIPTION OF THE INVENTION

The present invention involves the production of zeaxanthin using a microorganism, Flavobacterium multivorum, not heretofore known to produce this pigment. The invention involves isolating Flavobacterium multivorum cells and culturing them in a nutrient medium under conditions in which zeaxanthin is produced in recoverable amounts. The microbial cells can be separated from the fermentation liquor and used in biomass compositions.

Accordingly, one aspect of the present invention provides a process for the production of a zeaxanthin-containing composition comprising:

culturing a microorganism of Flavobacterium multivorum, or mutants or variants thereof, in a nutrient medium containing at least one assimilable carbon source, and at least one assimiable nitrogen source, wherein culturing of the microorganism results in the production of a culture comprising a zeaxanthin-containing cell mass and a residual nutrient medium; and recovering zeaxanthin from the culture.

According to a further aspect of the invention there is provided a feed composition comprising non-viable zeaxanthin-containing Flavobacterium multivorum cells, and residual cell debris and unused nutrient medium solids recovered from fermentation of Flavobacterium multivorum cells.

The term "Flavobacterium multivorum" is intended to include all microorganisms of the genus Flavobacterium and species multivorum that produce zeaxanthin, including their subcultures (mutants and variants), which cannot be definitely differentiated from the bacterial strain(s) described in the Examples or in Bergey's Manual (1984 Edition). Characteristics of F. multivorum are provided below, and a taxonomic description of F. multivorum strain AFB-44 is provided in Example 4. The term "mutants" as used herein includes mutants produced from Flavobacterium multivorum by various means, including but not limited to chemical mutagenic agents, ultraviolet radiation, phage exposure and the like. These processes and agents are well-known to practitioners in the art. Mutants described in this patent application were subjected to standard mutagenesis by NTG (n-methyl-N-nitro N-nitrosoguanidine) and to ultraviolet radiation, as described in Manual of Methods for General Bacteriology; ASM 1981, Chapter 13. The term "variants" as used herein includes variants of Flavobacterium multivorum including but not limited to those described in Bergey's Manual (1984 Edition). The term "biomass" is used herein to include non-viable zeaxanthin-containing Flavobacterium multivorum cells, residual debris, including unused media solids, recovered from fermentation of live Flavobacterium multivorum cells, and powders derived from this material. Said biomass may also include certain well-known additives, protectants, and emulsifiers and stabilized powders derived from this material. The biomass may be placed in an inert carrier containing, for example, starch and/or residual fermentation powder and/or one or more flours.

Flavobacterium multivorum grow in smooth, nonpigmented colonies of less that 1mm on a 1-day sheep blood agar plate; the plate shows no zones of inhibition surrounding penicillin, vancomycin, and polymyxin disks. The microorganisms are strongly oxidase-positive, catalase-positive, gram negative rods; non-motile; strongly sucrose-positive (most non-fermenting bacteria are sucrose-negative); always mannitol-negative and ethanol-negative; and urea-positive.

Producing zeaxanthin according to the present invention can be conducted using conventional culturing techniques well-known to practitioners in the art of producing zeaxanthin. See, for example, U.S. Patents 3,891,504; 3,841,967; 3,951,742; and 3,951,743. Briefly, Flavobacterium multivorum cells may be isolated in a known manner, the yellow cultures may be purified and subjected to cultivation on solid nutrient media or in liquid nutrient solutions under conditions in which zeaxanthin is produced intracellularly. The cell may be extracted with an organic solvent, and the zeaxanthin analyzed using spectrophotometry and high pressure liquid chromatography.

However, according to the present invention, the preferred process for producing zeaxanthin is shown in Example 2.

Preferably, the isolation and pure cultivation of the yellow Flavobacterium microorganisms of this invention is effected as follows. Material from a spring or other natural source, used as the source of the microorganisms, is suspended in physiological saline. Streak cultures are applied to Petri dishes. Then, the yellow colonies growing on the agar are examined for carotenoid content. Colonies of Flavobacterium multivorum can be identified by comparing the cells to the taxonomic description provided in Bergey's Manual (1984 Edition). Finally, the Flavobacterium multivorum microorganisms of this invention are identified by their zeaxanthin content [(confirmed by analytical procedures, preferably high performance liquid chromatography; F. Quackenbush, J. Liquid Chromatography, 10(4) pp. 643-653 (1987)] and isolated. Methods of determining zeaxanthin-producing microorganisms include, but are not limited to high performance liquid chromatography. Other processes for isolating F. multivorum are known to practitioners in the art, and are suitable for use in this invention. Strains of F. multivorum are also available through the American Type Culture Collection and various public and private depositories throughout the world.

The microorganisms employed in the present process can be cultivated in a nutrient medium in a conventional manner. In cultivating the microorganisms, many conventional solid nutrient media (or any conventional liquid nutrient solution media) which contain assimilable carbon or nitrogen can be utilized. The term "nutrient medium" designates a culture medium containing assimilable substances necessary for the growth of the microorganism. These substances include, in particular, a source of readily assimilable carbon and a source of readily assimilable nitrogen, and mineral salts, as noted below. The nutrient medium may also contain optional additives such as vitamins, pigment formation promoters, growth factors, certain inorganic salts such as NaCl (which are conventionally present in such media), and trace elements, as noted below.

However, according to the present invention, the preferred media has the following composition (percentages are by weight of total medium):

| | |
|---|---|
| Nitrogen source | 3-10% |
| Carbon source | 1-7% |
| Minerals | .0001-0.5% |
| Na₂HPO₄ | 0-1% |
| Fat source | .5-3% |
| Growth factor(s) | 0-1% |
| Enzyme | .001-.1% |
| Water | remainder |

Sources of readily assimilable nitrogen include but are not limited to numerous substances of animal, vegetable and/or microbial origin as well as inorganic nitrogen compounds. Among the preferred sources of readily assimilable nitrogen are soya meal, fish meal, meat meal, meat extract, peptone, corn steep liquor, yeast extract, amino acids, ammonium salts (such as ammonium phosphate or ammonium sulfate), and protein hydrolysates, in particular products obtained by the acidic or enzymatic hydrolysis of vegetable proteins such as soybeans, soya, or peanut proteins and/or the casein hydrolysate called tryptone. The nitrogen source may also contain a material prepared by acidic or enzymatic hydrolysis of a biomass recovered as a byproduct in the biosynthesis of a carotenoid pigment by culture of a bacterium of the Flavobacterium genus, in particular by the hydrolysis of a biomass of Flavobacterium cultured for the production of zeaxanthin and from which the pigment has been extracted.

The most preferred assimilable nitrogen source is corn steep liquor, because of its low cost and the presence of desirable growth factors.

Sources of readily assimilable carbon include, but are not limited to sugars and their polymers, such as starches, dextrin, saccharose, maltose, lactose, glucose, and molasses; fatty acids; and polyalcohols, such as glycerine. Preferred carbon sources include corn, corn flour, rice, milo, wheat, starch, lactate, acetate, and glucose feed. Corn flour is most preferred because of its price, particle size, and culture use at levels ranging from 1-7% by weight of the total medium. As is evident to one skilled in the art, corn, corn flower, and starch require treatment with a starch liquefaction enzyme, such as α-amylase (commercially available as Termamyl 120L), which hydrolyzes starch to dextrin. Without this treatment, the starch would set up into a solid mass upon heating. However, too much enzymatic hydrolysis reduces yields, while too little hydrolysis reduces yields and increases fermentation times.

The nutrient media may also contain trace elements originating from present or added mineral or organic ingredients. For example, sulphur and phosphorus can originate from inorganic or organic ingredients present in the nutrient medium, or they can be specifically added to the nutrient medium. If desired or required, growth-promoting agents or stimulants such as, for example, vitamins, can also be added to the nutrient medium. The preferred minerals are low levels of ferrous sulfate (which improves cell growth) and disodium phosphate.

The fat source includes but is not limited to soapstock, soybean oil, sunflower oil, and olive oil; soapstock is preferred.

Cultivation is preferably carried out by employing certain growth factors or yield-promoting additives in the media. The preferred growth factor is yeast extract.

The strains utilizable for the cultivation process can be introduced to the fermenting vessel from the streak culture plate according to known methods. The preferred methods are via the agarslant culture and glass-flask liquid culture.

Cultivation of the microorganism under conditions which lead to the formation of zeaxanthin according to the process of this invention may be carried out in any conventional manner. In accordance with a preferred embodiment of carrying out this process, cultivation occurs in an aqueous medium. In carrying out this submerged cultivation, any conditions which are conventionally utilized in carrying out submerged cultivation may be used. In the preferred process, the fermentation is carried out at a temperature between 10° and 35° C., at a pH range of about 6.5 to about 8.0, and for about 24 to about 72 hours. The preferred conditions for the cultivation process using the nutrient media of the present invention are a temperature between about 22° C and about 30° C, a pH of about 7.2, and a cultivation period of about 30-36 hours.

The pH of the culture medium is adjusted between 6.5 and 8.0, preferably between 7.0 and 7.5. The adjustment of pH may be effected with substances including, but not limited to aqueous solutions of sodium hydroxide, potassium hydroxide or ammonium hydroxide. These substances are well-known to practitioners in the art.

In the process of this invention, formation of pigment increases in proportion to the growth of the culture, with the maximum pigment formation being obtained in about 30-36 hours.

After the culture time is completed, the zeaxanthin content of the fermentation medium can be determined. For this purpose, the biomass is separated from the nutrient substrate by centrifugation and the zeaxanthin is extracted from the cells. The zeaxanthin content of the extraction solution can be determined colorimetrically by comparison with standard solutions of synthetic zeaxanthin in the same solvent.

At the end of culture, the fermentation broth may be concentrated and the zeaxanthin extracted from the cells, using a polar organic solvent, including but not limited to, acetone, hexane, or a chlorinated solvent such as chloroform, or by supercritical fluid extraction. See, for example, F. Favati, et al., J. Food Sci. 53(5): 1532-1535 (1988).

Alternatively, the biomass may be separated from the culture medium, for example by centrifugation, decantation or filtration. If centrifugation is used, cell recovery may be improved by the addition of bentonite and calcium chloride (see Example 2). In the preferred method, bentonite and calcium chloride are added to the fermentation broth, which is then heated in order to kill any viable cells. The broth is then centrifuged in order to recover a paste of packed cells and unused media solids. The cell paste may be slurried in water to a workable viscosity. EDTA (about .2%), BHA (about .05%), Tween® (about 0.1%), and tocopherol acetate (about .015%) may be added to the slurry in order to prevent or reduce pigment breakdown; the percent used is based on cell weight in the slurry. The slurry is then homogenized (using, for example, glass bead cell rupture or a high pressure homogenizer) and dried for future use. The preferred method of drying is by spray drying.

The biomass may be used as an additive in chicken feeds, for example, or it may also be extracted with a polar organic solvent, as noted above.

The pigment-containing biomass can be advantageously utilized in accordance with this invention to color foodstuffs without the necessity for isolating pure zeaxanthin pigment. On the other hand, the intracellular zeaxanthin can be separated from the cells in a conventional manner. A preferred method of separating or extracting the zeaxanthin involves carefully drying the cell-mass; pulverizing the dried cell-mass; digesting the pulverized material with an inert organic solvent; filtering the solution; and isolating the pure zeaxanthin by elution of the filtration residue with an inert organic solvent. The individual steps of the preferred method can be carried out in a conventional manner. According to a particularly preferred method of separating the zeaxanthin, the cell-mass is dried by spray drying. Inert organic solvents include but are not limited to a lower alkanol, preferably ethanol; a ketone, preferably acetone; or a halogenated hydrocarbon; preferably chloroform. Further, particularly preferred is to take up the evaporation residue in ethyl acetate, a lower alkanol or mixtures thereof. Further, particularly preferred is filtering the solution over silica gel, neutral aluminum oxide, or magnesium silicate. Still more preferred is eluting the zeaxanthin with a chlorinated hydrocarbon, particularly methylene chloride or dichloroethylene or a di-lower alkyl ether, particularly diethyl ether. Alternatively, the dried powder may be extracted using supercritical extraction with CO₂ gas under high pressure.

The pigment formed by Flavobacterium consists of up to 95-99 percent of zeaxanthin. Tests show that Flavobacterium-produced zeaxanthin is identical to zeaxanthin isolated from Zea mais.

The almost pigment-free cell-mass remaining after extraction of the zeaxanthin can be used as an ideal source of proteins (essential amino acids such as methionine and lysine) and vitamins (especially vitamins of the B-group and particularly vitamin B₁₂) for the raising of poultry.

The present invention also includes mutants and variants of F. multivorum having substantially the same taxonomic characteristics as the AFB-44 strain shown in Example 4. Mutations may be induced by conventional techniques, as noted above.

All publications mentioned in this specification are indicative of the level of skill of those skilled in the art to which this invention pertains.

Although the foregoing invention has been described in some detail by way of illustration and example for purposes of clarity of understanding, it will be obvious that certain changes and modifications may be practiced within the scope of the appended claims.

### EXAMPLES

### Example 1. Isolation of F. Multivorum.

Samples from the environment were screened for yellow-orange pigmented bacteria by streaking the samples directly onto an agar media petri plate (2.0% agar, 0.02% trypticase soy broth, 0.02% yeast extract and 0.25% sodium chloride). In addition, samples were incubated with illumination in water-filled whirl-pack bags for 3 days at room temperature as an enrichment procedure (with 0.5% sodium chloride) prior to streaking on agar.

Pigmented strains from a number of culture collections from around the world were maintained on trypticase soy agar + 1% yeast extract slants. The described F. multivorum strain AFB-44 was isolated from a fresh water spring near High Ridge, Missouri.

Hundreds of strains isolated in this manner or obtained from culture collections were then inoculated into a pigment screening broth (autoclaved at 121°C; 30 minutes):

| | |
|---|---|
| Trypticase soy broth | 1.0% |
| Pyridoxine | 0.001% |
| Casitone | 0.25% |
| Disodium phosphate | 1.0% |
| Sodium caseinate | 0.25% |
| Yeast extract | 0.25% |
| Corn steep liquor | 0.1% |
| methionine | 0.1% |
| Ferrous sulfate | 0.01% |
| mg⁺⁺ sulfate | 0.01% |
| mn⁺⁺ sulfate | 0.01% |
| Zn⁺⁺ sulfate | 0.01% |
| thiamine | 0.01% |
| glycerol | 0.5% |
| | |
| pH | 7.8 |

Cultures were incubated at 25°C (with illumination) for 72 hours or until sufficient growth occurred, and were harvested by pelleting with centrifugation (25,000 rcf). They were grown as 50 ml batches in 300 ml dimpled flask at 250 rpm on a gyratory incubator/shaker. Pellets were frozen and then extracted with acetone. Centrifuged extracts were then dried and brought up in hexane and applied to alumina chromatograph apparatus and developed per the AOAC method for xanthophylls [Quackenbush et al, J. Assoc. Off. Anal. Chem. 56: 748-753 (1973)]. Various fractions were collected, particularly dihydroxycarotenoids, and were scanned for absorption maxima by a Beckman® scanning spectrophotometer.

Out of thousands of starting isolates, 12 isolates produced a similar absorption spectra and chromatographic retention to that reported for zeaxanthin. These isolates were then grown, extracted and submitted for authentication to an outside expert by reverse phase HPLC [Quackenbush F., J. Lig. Chrom. 10(4): 643-653 (1988)]. One culture produced a relatively pure yield of zeaxanthin as its major pigment (95%).

This culture was submitted in confidence to two independent laboratories for a taxonomic work-up. Both laboratories concluded that the culture was not similar to previously describe zeaxanthin-producing bacteria. The culture strain was characterized as a Flavobacteriumium multivorum strain. This was the first report of zeaxanthin production by this species.

### Example 2. Cultivation of Flavobacterium multivorum.

The culture is maintained on a slant tube of Plate Count Agar. These slants are inoculated and then incubated for 24 hours at 30°C. Stock slants are stored in a refrigerator for use as inoculum for liquid media.

The liquid media has the following composition:

| | |
|---|---|
| Corn Steep liquor | 7.0% |
| Corn Flour | 5.0% |
| FeSO₄ | .001% |
| Soapstock | 1.0% |
| Yeast Extract | .05% |
| Na₂HPO₄ | .1% |
| Termamyl 120L | .01% |
| Water | 86.839% |
| | $\overline{\text{100.00%}}$ |

The nutrient media was adjusted to a pH of 7.6 using concentrated (10N) sodium hydroxide. The media was then heated to about 85°C and held for 30 minutes; this allows enzymatic hydrolysis of the starch into a dextrin. The heated media was then sterilized at 121°C for 30 minutes. After cooling, the media is inoculated from a stock slant (as prepared above), or from a previously inoculated broth culture which is about 24 hours old. The level of inoculum is about 5% V/V.

The growth conditions for the inoculated nutrient media were a temperature at 30°C; pH was 7.2; time was 36 hours; the amount of inoculum was 5% by weight; and under continuous aeration. Aeration is 200 rpm in a shaker incubator using 300 ml dimpled erlenmeyer flasks with 30 ml or media, or, in a fermentor stirring at 400 rpm, bubbling air at a rate sufficient to maintain a dissolved oxygen level of 50% of saturation. In this experiment, the bubbling rate was about 0.25 VVM.

At approximately 12 hours into the fermentation, lipase and glucoamylase was added (at low levels, about 0.03% by weight of the media, or 30 lipase units and 6 AG units per liter of fermentation media).

After completion of fermentation, Flavobacterium multivorum cells were harvested from the medium by centrifugation after the addition of .006 g/l to 0.01 g/l bentonite and 0.16 g/l to 0.4 g/l CaCl₂. The medium is then heated to 50°C in order to kill any viable cells, then the broth is centrifuged in order to recover a thick paste of packed cells and unused media solids. The cell paste is then reslurried, and EDTA (0.2%), tocopherol acetate (0.015%), BHA (0.05%), and Tween® 80 (0.1%) are added to slurry. The slurry is then homogenized and dried for future use. This dried product was then used for the layer and broiler feeding studies. The dried biomass was stored for two months at room temperature to simulate a storage stability test.

### Example 3

The following experiment was done to demonstrate the advantages of our described process versus growth on other media described in the literature and patents.

Media with the following compositions were formulated, autoclaved, cooled and inoculated with a strain of F. multivorum, as noted.

Media A, B, and C are media described in the patents noted in the Relevant Literature section. Media D and E are nutrient media of the present invention.

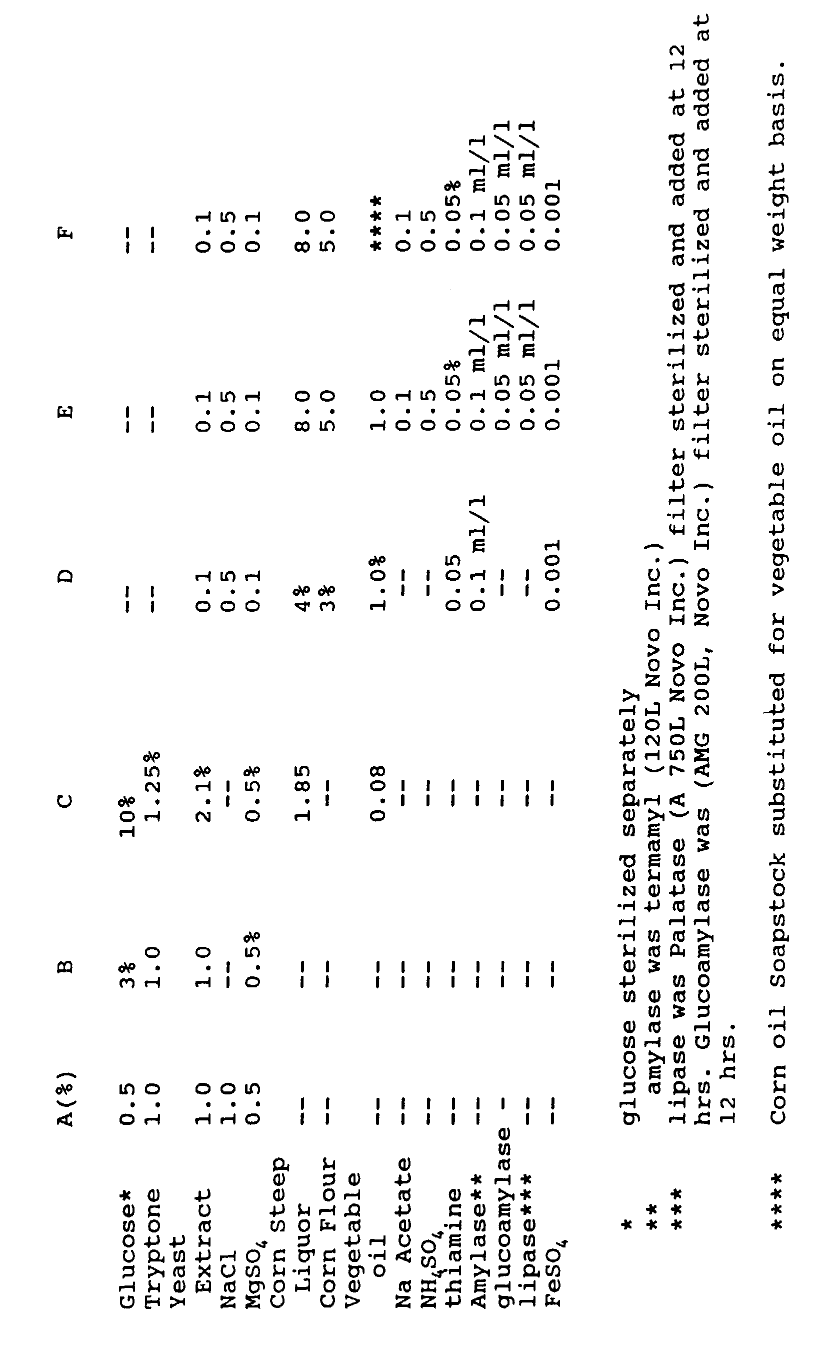

All pH's were adjusted to 7.5 and maintained by periodic checking. Only media C required significant adjustments. All media were autoclaved at 121°C for 30 minutes except D and E which additionally were held at 80-90°C for 30 minutes just prior to sterilization. All media except E and F were inoculated at 5% volume/volume with the AFB 44 strain and incubated on a rotary shaker at 250 rpm for 24 hours at 30°C and then 24 hours at 26°C prior to centrifugal harvesting. Media E (with mutant) and F were inoculated separately with a mutant derived from the AFB-44 strain and in this case the mutant grown in media E and F were incubated at 30°C for just 32 hours. The results are shown in the following table.

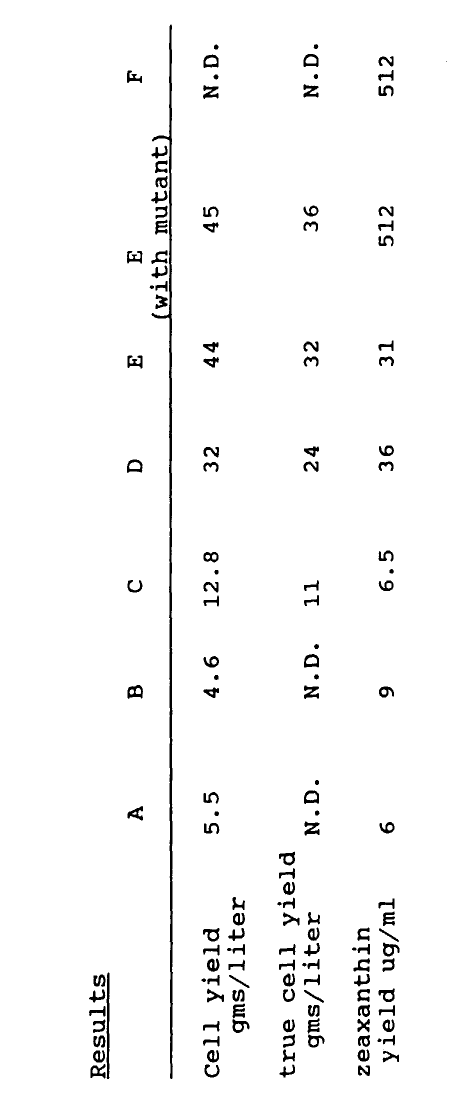

Cell yields were calculated by centrifuging at 20,000 rcf for 10 minutes, decanting the supernatant, and drying the resulting pellet at 105°C for 24 hours. True cell yields were calculated by first centrifuging at 3,000 rcf to remove fermentation insolubles, and drying this pellet; the dry weight of the pellet was subtracted from the regular cell yield values. Zeaxanthin was calculated by extracting the frozen cell pellets with acetone and then reading the O.D.₄₅₀ on a spectrophotometer of the centrifuged extracts. These readings were then multiplied by appropriate factors for extinction coefficient and dilution effects.

Additionally, media D was used to culture other Flavobacterium multivorum strains in order to show that the nutrient media of the present invention works with other F. multivorum strains, and to show that the nutrient media leads to improved zeaxanthin yield.

Media D was inoculated with strains K2361 or K1180 (described in Example 4), and cultivated as shown in Example 2. K2361 produced a crude cell yield of 25 g/l, a true cell yield of 13 g/l, and 11 µ/ml zeaxanthin. K1180 produced a crude cell yield of 24.5 g/l, a true cell yield of 12.5 g/l, and 4 µ/ml zeaxanthin. The results for K2361 show that the nutrient media of the present invention improves the cell yield and amount of zeaxanthin produced by some strains of F. multivorum. The results for K1180 show that the more economical nutrient media of the present invention produces about the same cell yield and amount of zeaxanthin as more expensive nutrient media.

The results of these experiments demonstrate that with the AFB-44 strain of F. multivorum:
a) media D and E (the present invention) produce significantly greater cell yields and zeaxanthin yields than the media A, B, and C described in previous patent literature.
b) although media C and E have approximately equal solids initially, media E provides a better yield of cells and pigment.
c) while media A, B, and C are very expensive relative to D and E, the latter produced better yields.
d) by using mutants of the AFB-44 strain adapted to media E, very high cell and pigment yields could be demonstrated in production times as low as 32 hours (i.e., more rapid growth). AFB-44 mutants in media E resulted in the highest cell and pigment yields yet established.
e) soapstock can be substituted for vegetable oil while retaining equal zeaxanthin yield.

### Example 4. Comparison to Other Flavobacterium Microorganisms.

An isolate of Flavobacterium multivorum (AFB-44) was compared taxonomically to other Flavobacterium species: ATCC 21588 (or 21588L) and ATCC 11947 are described in U.S. Patent 3,841,967; 3,951,742; and 3,951,743. ATCC 21081 is disclosed in U.S. Patent 3,891,504 and 3,841,967. It should be noted that the patent strains, ATCC 21588 and 21081, would not be classified in the Flavobacterium genus according to the new taxonomic scheme described in the 1984 edition of Bergey's Manual.

ATCC No. 21081 is significantly different from the AFB-44 strain in its growth temperature requirements and its absolute requirement for salt.

ATCC No. 21588 is significantly different from F. multivorum in many fermentation and utilization patterns; it is significantly different in that F. multivorum strains grow well at 35°C and in motility-nitrate agar and infusion broth, while ATCC 21588 does not. In addition, ATCC 21588 is sensitive to penicillin and vancomycin, while F. multivorum is not. ATCC 21588 was also LANA negative and could produce spore like structures; both attributes are uncharacteristic of the Flavobacterium genus.

The taxonomy work was carried out by a recognized expert in pigmented bacteria taxonomy. His conclusions that the F. multivorum strain is significantly different than ATCC 11947, 21081, or 21588 was supported by a similar conclusion from the microbial taxonomy service of the American Type Culture Collection.

| Acid from | No. 21588 | No. AFB-44 |
|---|---|---|
| adonitol | - | - |
| L-arabinose | + | + |
| cellobiose | + | + |
| ethanol | - | - |
| fructose | + | + |
| galactose | + | + |
| glucose | + | + |
| inositol | + | - |
| lactose | + | + |
| maltose | + | + |
| mannitol | + | - |
| melibiose | N.D. | N.D. |
| raffinose | + | + |
| rhamnose | - | +w |
| sorbital | - | - |
| sucrose | + | + |
| xylose | - | + |
| D-arabinose | - | + |
| salicin | - | + |
| trehalose | + | + |

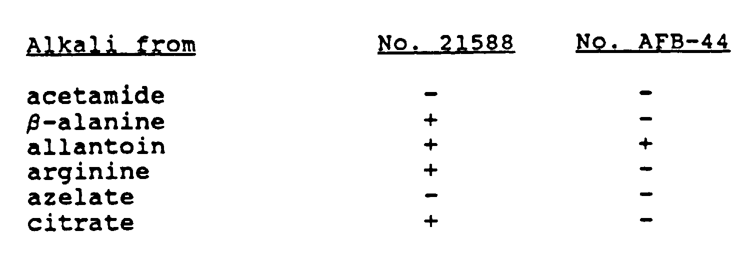

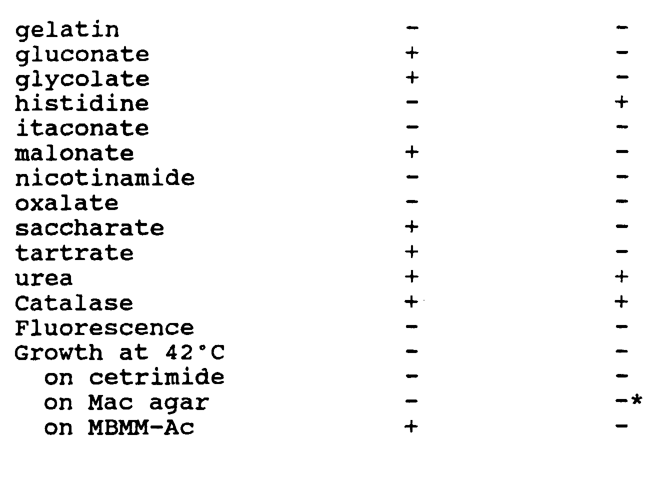

| Hydrolysis of | No. 21588 | No. AFB-44 |
|---|---|---|
| casein | - | - |
| DNA | - | -w |
| esculin | +w | + |
| gelatin | - | - |
| starch, Argo | - | - |
| Tween® 80 | - | + |
| Indole | - | - |
| KOH test | ?- | + |
| LANA test | - | + |
| Lysine (LDC) | - | - |
| Motility | - | - |
| Nitrate - gas | - | - |
| - nitrate | - | - |
| Zn test | + | + |
| Nitrate - gas | - | - |
| Ornithine (ODC) | - | - |
| Oxidase | + | + |
| Phenylalanine (PPA) | - | - |
| | | |

| Sensitive to | No. 21588 | No. AFB-44 |
|---|---|---|
| penicillin (2U) | + | - |
| polymyxin B (300U) | + | - |
| vancomycin (5µg) | + | - |

| | ATCC #21081 | AFB-44 |
|---|---|---|
| Growth on: | | |
| TSA, RT | nil | good |
| TSA, 30°C | nil | good |
| PYEPO, RT | good | nil |
| PYEPO,* 30°C | nil | nil |

| | | |
|---|---|---|
| * Marine agar | | |

ATCC No. 21081 is a gram-negative rod, and non-motile in a wet mount. Its growth characteristics are not those of genus Flavobacterium as presently defined by Holmes et al in Bergey's Manual 1984, but are compatible with some taxonomy of Weeks' Flavobacterium Section II in Bergey's Manual 1974. It has an obligable requirement for NaCl and cannot grow at 30°C.

### Example 5 - Comparison of F. Multivorum Strains

The original AFB-44 strain, after isolation as previously described, was identified as a zeaxanthin-producing bacterium. After initial comparisons with known Flavobacterium strains, it was determined that it was uniquely different and that it should be categorized as F. multivorum. During the initial extensive screening procedure, this was the only Flavobacterium strain that could be identified to produce zeaxanthin. This included the entire collections of the Flavobacteriumium/Cytophaga group from five public collections from around the world. We concluded that zeaxanthin-producing pigmented bacteria were very rare. After identification of our organism, it was determined that additional F. multivorum strains should be checked. F. multivorum strains available from the UCLA-Microbiology Department collection, Nos. K-1213, K-1204, K-1180, K-2361, K-2303, were compared with strain AFB-44, as follows. The strains were grown on PCA slants and inoculated into a liquid media and grown at 30°C for 24 hours and 24°C for 48 hours on a reciprocal shaker in dimpled flasks prior to harvesting by centrifugation. In this experiment, illumination was also used. Samples were extracted with acetone from the frozen pellets and the extract was dried under N₂ gas and brought back up in hexane and applied to alumina (neutral) column chromatography apparatus. Increasing acetone/hexane ratios were used to fractionate; spectrophotometric scans were used to check absorption maxima of each fraction. The following results were obtained.

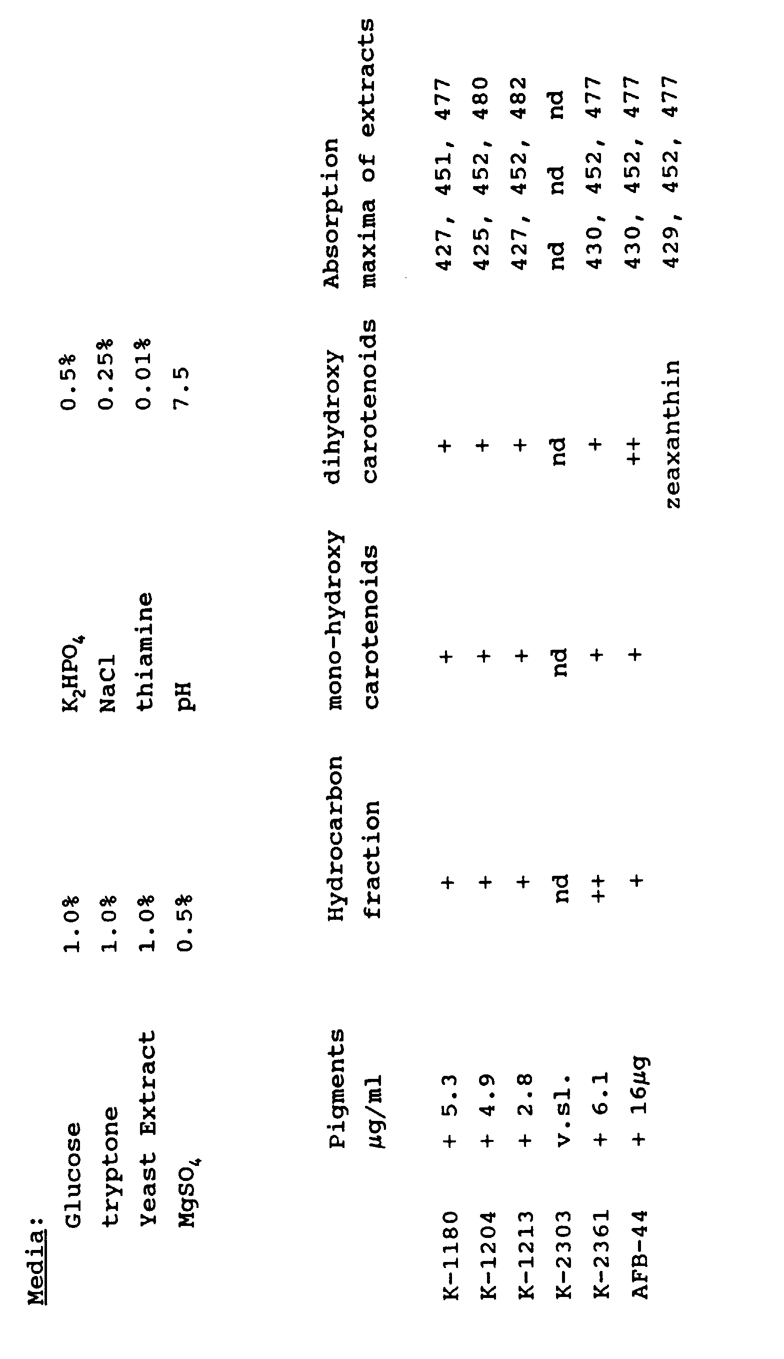

The data demonstrated that:
a) other strains of F. multivorum, surprisingly, produce zeaxanthin.
b) it appears that every strain of F. multivorum produces a quantifiable amount of pigment (however, in this experiment, K-2303 could not be shown to produce quantifiable pigment, i.e., is a weak pigmenter, and the production of zeaxanthin could not be determined).
c) the wild strain of AFB-44 produces more pigment and a better zeaxanthin/total carotenoids ratio than the other strains.
d) column chromatography shows that the dihydroxy carotenoid produced by all of the strains (except K-2303) appears to be primarily zeaxanthin.
e) that publicly available F. multivorum strains produce zeaxanthin.
f) that a heretofore unknown grouping of Flavobacterium share a propensity towards zeaxanthin as a common pigment of their cell composition.

### Example 6. Feeding studies demonstrating increased biological availability, stability and pigmenting power of a biomass composition relative to current commercial processed pigment sources.

A biomass composition, which had been prepared as shown in Example 2, and had been stored at room temperature for two months was fed to broiler chickens and compared to stabilized and saponified extracts of marigold flowers. The basal diet was as follows:

| Ingredient | Starter | Grower (Wheat) |
|---|---|---|
| Wheat | 45.32 | 61.46 |
| Corn | 10.00 | 10.00 |
| Soybean Meal | 31.35 | 14.32 |
| Poultry Meal | 2.40 | 6.00 |
| Poultry Fat | 7.28 | 5.31 |
| Limestone | 1.24 | 1.03 |
| Decal. Phos. | 1.44 | 0.96 |
| Salt | 0.40 | 0.30 |
| Choline | 0.20 | 0.20 |
| Trace Mineral | 0.10 | 0.10 |
| Vitamin Premix | 0.05 | 0.05 |
| Lysine | --- | 0.12 |
| DL-Methionine | 0.18 | 0.10 |
| Coccidiostat | 0.05 | 0.05 |
| | | |

| Nutrients | | |
|---|---|---|
| Protein, % | 23.00 | 19.00 |
| Energy (Kcal/1b) | 1450 | 1450 |
| Calcium, % | 1.00 | 0.90 |
| Total Phos, % | 0.70 | 0.65 |
| TSAA, % | 0.93 | 0.75 |
| Lysine, % | 1.26 | 1.00 |

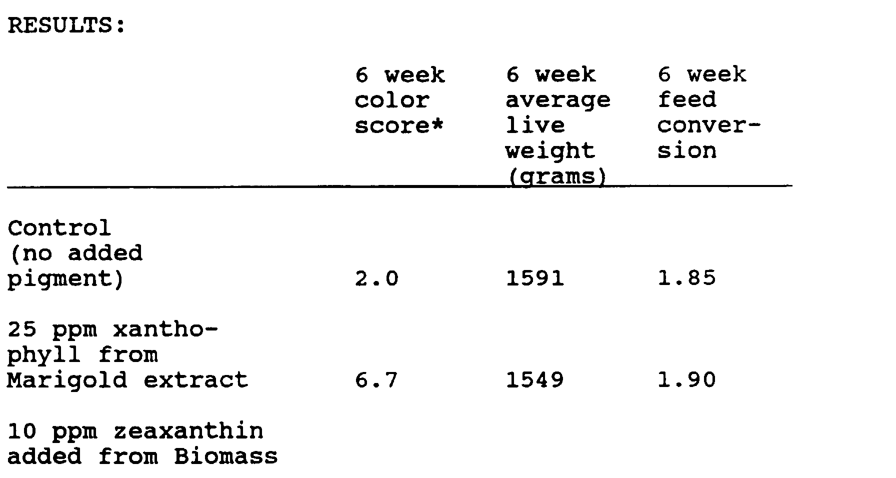

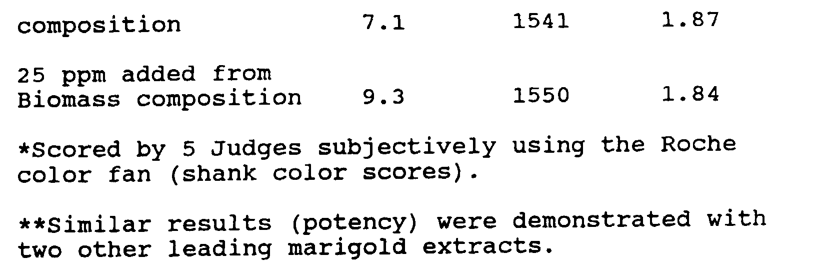

The results show no deleterious effects on the chickens and that 10 ppm of xanthophyll from the biomass composition was biologically available and equal to 25 ppm of xanthophyll from marigold extract the best natural stabilized and processed commercial natural source of xanthophylls. The xanthophyll must be biologically available to show improved pigmentation and also appears to pigment more readily. The results demonstrate a microbial pigment composition that surprisingly had good stability and biological availability without the need for expensive extraction and saponification processes. In addition, no harmful effects can be seen in the growth data.

## Claims

1. A process for the production of a zeaxanthin-containing composition comprising:
culturing a microorganism of Flavobacterium multivorum, or mutants or variants thereof, in a nutrient medium containing at least one assimilable carbon source, and at least one assimilable nitrogen source, wherein culturing of the microorganism results in the production of a culture comprising a zeaxanthin-containing cell mass and residual nutrient medium; and recovering zeaxanthin from the culture.

2. A process according to claim 1 further comprising separating the zeaxanthin containing cell mass from the nutrient medium.

3. A process according to claim 2 further comprising separation of the zeaxanthin from the cell mass.

4. A process according to claim 1 wherein the zeaxanthin is recovered from the culture as a cell paste.

5. The process of any one of claims 1 to 3 wherein said Flavobacterium multivorum, or a variant or mutant thereof, has the following taxonomic characteristics: penicillin-insensitive, vancomycin-insensitive, and polymyxin-insensitive; strongly oxidase-positive, catalase-positive, gram negative rods; non-motile; strongly sucrose-positive; mannitol-negative; and ethanol-negative; urea-positive; and non-proteolytic.

6. The process of any one of claims 1 to 3 wherein said process yields at least 40g/l of zeaxanthin-containing cells.

7. The process of any one of claims 1 to 3 wherein said nutrient medium contains corn steep liquor or other assimilable nitrogen sources, and an enzyme-treated carbon source.

8. The process of claim 7 wherein said enzyme is α-amylase.

9. The process of any one of claims 1 to 3 wherein the glucose level in said media is less than about 0.035% by weight.

10. The process of any one of claims 1 to 3 wherein said assimilable carbon source is selected from the group consisting of corn, rice, milo, wheat, flours derived from these grains, starches derived from these grains, malto-dextrin, and glucose.

11. The process of any one of claims 1 to 3 wherein said assimilable nitrogen source is selected from the group consisting of corn steep liquor, hydrolyzed casein, hydrolyzed soybeans, ammonium phosphate, and ammonium sulfate, ammonia, yeast extract, a protein hydrolysate and a hydrolysate of a Flavobacterium biomass.

12. The process of any one of claims 1 to 3 wherein said culturing step is conducted in a temperature range of about 16°C to 38°C, at a pH range of about 6.0 to about 8.5, and for about 24 to about 72 hours.

13. The process of claim 12 wherein said process is substantially complete in about 32 hours.

14. The process of any one of claims 1 to 3 wherein said nutrient medium is an aqueous solution.

15. The process of any one of claims 1 to 3 wherein said cultivation is conducted between about 22°C and about 34°C.

16. The process of any one of claims 1 to 3 wherein said cultivation is conducted at a pH range of about 6.5 to about 8.0.

17. The process of any one of claims 1 to 3 wherein said cultivation is conducted in about 24 to about 72 hours.

18. The process of any one of claims 1 to 3 wherein said process is substantially complete in about 32 hours.

19. The process of any one of claims 1 to 3 further comprising adding enzymes to said nutrient medium during cultivation.

20. The process of claim 19 wherein said enzyme is glucoamylase, lipase, or mixtures thereof.

21. The process of any one of claims 1 to 3 wherein a promoter of pigment formation is added to said nutrient media.

22. The process of claim 3 wherein the zeaxanthin is separated from said cell-mass by;
digesting said cell-mass with an inert organic solvent to dissolve the zeaxanthin; and
removing said solvent from the zeaxanthin.

23. The process of claim 22 wherein the solvent is removed from the zeaxanthin by evaporation.

24. The process according to claim 1, wherein said nutrient medium contains about 1% by weight to about 7% by weight of assimilable carbon source and from about 3% by weight to about 10% by weight of assimilable nitrogen source.

25. A process according to claim 1, in which, following the culturing step, zeaxanthin is solvent extracted from the microorganism cells.

26. The process of claim 1 wherein additionally comprising separating said cell mass from said fermentation liquor to form a cell paste;
slurrying said paste;
homogenizing said paste; and
drying said paste.

27. The process of claim 26 wherein said paste is slurried in protectants and an emulsifier.

28. The process of claim 27 wherein said protectants are antioxidants, chelating agents, or mixtures thereof.

29. The process of claim 26 wherein the assimilable carbon source is enzyme-treated.

30. The process of claim 1 wherein the assimilable carbon source is selected from the group consisting of corn, rice, milo, wheat, flours derived from these grains, starches derived from these grains malto-dextrin, flours and starches, and starches derived from these grains, and glucose; and said assimilable nitrogen source is selected from the group consisting of corn steep liquor, hydrolyzed casein, hydrolyzed soybeans, ammonium phosphate, ammonium sulfate, ammonia, yeast extract, a protein hydrolysate and a hydrolysate of Flavobacterium biomass.

31. A feed composition comprising non-viable zeaxanthin-containing Flavobacterium multivorum cells, and residual cell debris and unused nutrient medium solids recovered from fermentation of Flavobacterium multivorum cells.

32. The composition of claim 31 which is a poultry feed composition, salmonoid feed composition, or food colouring composition.

33. A feed composition comprising zeaxanthin-containing Flavobacterium multivorum cells and a nutrient medium.

34. The composition of claim 33 wherein said composition is a poultry feed composition.

35. The use of Flavobacterium multivorum cells in the manufacture of a feed composition.

## Patentansprüche

1. Verfahren zur Herstellung einer Zeaxanthin enthaltenden Zusammensetzung, umfassend:
das Kultivieren eines Flavobacterium multivorum-Mikroorganismus oder von Mutanten oder Varianten davon in einem Nährstoffmedium, das zumindest eine Quelle für assimilierbaren Kohlenstoff und zumindest eine Quelle für assimilierbaren Stickstoff enthält,
worin das Kultivieren des Mikroorganismus zur Herstellung einer Kultur führt, die eine Zeaxanthin enthaltende Zellmasse und restliches Nährstoffmedium enthält,
sowie das Gewinnen von Zeaxanthin aus der Kultur.

2. Verfahren nach Anspruch 1, das weiters das Abtrennen der Zeaxanthin enthaltenden Zellmasse vom Nährstoffmedium umfaßt.

3. Verfahren nach Anspruch 2, das weiters das Abtrennen von Zeaxanthin aus der Zellmasse umfaßt.

4. Verfahren nach Anspruch 1, worin das Zeaxanthin aus der Kultur als Zellpaste gewonnen wird.

5. Verfahren nach einem der Ansprüche 1 bis 3, worin das Flavobacterium multivorum oder eine Variante oder Mutante davon folgende taxonomische Eigenschaften aufweist: penicillinunempfindlich, vancomycinunempfindlich und polymyxinunempfindlich; stark oxidasepositive, katalasepositive, gramnegative Stäbchen; nicht beweglich; stark saccharosepositiv, mannitnegativ; und ethanolnegativ; harnstoffpositiv; und nichtproteolytisch.

6. Verfahren nach einem der Ansprüche 1 bis 3, worin das Verfahren zumindest 40 g/l Zeaxanthin enthaltende Zellen ergibt.

7. Verfahren nach einem der Ansprüche 1 bis 3, worin das Nährstoffmedium Maisquellwasser oder andere Quellen für assimilierbaren Stickstoff und eine enzymatisch behandelte Kohlenstoffquelle enthält.

8. Verfahren nach Anspruch 7, worin das Enzym α-Amylase ist.

9. Verfahren nach einem der Ansprüche 1 bis 3, worin der Glukosespiegel in den Medien geringer als etwa 0,035 Gew.-% ist.

10. Verfahren nach einem der Ansprüche 1 bis 3, worin die Quelle für assimilierbaren Kohlenstoff aus der Gruppe ausgewählt ist, die aus Mais, Reis, Milo, Weizen, aus diesen Getreiden gewonnenen Mehlen, aus diesen Getreiden gewonnenen Stärken, Maltodextrin und Glukose besteht.

11. Verfahren nach einem der Ansprüche 1 bis 3, worin die Quelle für assimilierbaren Stickstoff aus der Gruppe ausgewählt ist, die aus Maisquellwasser, hydrolysiertem Kasein, hydrolysierten Sojabohnen, Ammoniumphosphat und Ammoniumsulfat, Ammoniak, Hefeextrakt, einem Proteinhydrolysat und einem Hydrolysat einer Flavobacterium-Biomasse besteht.

12. Verfahren nach einem der Ansprüche 1 bis 3, worin der Kultivierungsschritt in einem Temperaturbereich von etwa 16 bis 38°C, in einem pH-Bereich von etwa 6,0 bis etwa 8,5 und etwa 24 bis etwa 72 h lang durchgeführt wird.

13. Verfahren nach Anspruch 12, worin das Verfahren nach etwa 32 h im wesentlichen abgeschlossen ist.

14. Verfahren nach einem der Ansprüche 1 bis 3, worin das Nährstoffmedium eine wäßrige Lösung ist.

15. Verfahren nach einem der Ansprüche 1 bis 3, worin das Kultivieren zwischen etwa 22°C und etwa 34°C durchgeführt wird.

16. Verfahren nach einem der Ansprüche 1 bis 3, worin das Kultivieren in einem pH-Bereich von etwa 6,5 bis etwa 8,0 durchgeführt wird.

17. Verfahren nach einem der Ansprüche 1 bis 3, worin das Kultivieren in etwa 24 bis etwa 72 h durchgeführt wird.

18. Verfahren nach einem der Ansprüche 1 bis 3, worin das Verfahren nach etwa 32 h im wesentlichen abgeschlossen ist.

19. Verfahren nach einem der Ansprüche 1 bis 3, das weiters das Zugeben von Enzymen zum Nährstoffmedium während des Kultivierens umfaßt.

20. Verfahren nach Anspruch 19, worin das Enzym Glukoamylase, Lipase oder ein Gemisch daraus ist.

21. Verfahren nach einem der Ansprüche 1 bis 3, worin den Nährstoffmedien ein Pigmentbildungsverstärker zugegeben wird.

22. Verfahren nach Anspruch 3, worin das Zeaxanthin von der Zellmasse durch
Digerieren der Zellmasse mit einem inerten organischen Lösungsmittel zum Auflösen des Zeaxanthins; und
Entfernen des Lösungsmittel vom Zeaxanthin abgetrennt wird.

23. Verfahren nach Anspruch 22, worin das Lösungsmittel durch Verdampfen vom Zeaxanthin abgetrennt wird.

24. Verfahren nach Anspruch 1, worin das Nährstoffmedium etwa 1 Gew.-% bis etwa 7 Gew.-% Quelle für assimilierbaren Kohlenstoff und von etwa 3 Gew.-% bis etwa 10 Gew.-% Quelle für assimilierbaren Stickstoff enthält.

25. Verfahren nach Anspruch 1, bei dem nach dem Kultivierungsschritt Zeaxanthin durch Lösungsmittelextraktion von den Mikroorganismuszellen extrahiert wird.

26. Verfahren nach Anspruch 1, das zusätzlich
das Abtrennen der Zellmasse von der Fermentationsflüssigkeit zur Bildung einer Zellpaste;
das Aufschlämmen der Paste;
das Homogenisieren der Paste; und
das Trocknen der Paste umfaßt.

27. Verfahren nach Anspruch 26, worin die Paste in Schutzmitteln und einem Emulgator aufgeschlämmt wird.

28. Verfahren nach Anspruch 27, worin die Schutzmittel Antioxidantien, Chelatbildner oder Gemische daraus sind.

29. Verfahren nach Anspruch 26, worin die Quelle für assimilierbaren Kohlenstoff enzymatisch behandelt ist.

30. Verfahren nach Anspruch 1, worin die Quelle für assimilierbaren Kohlenstoff aus der Gruppe ausgewählt ist, die aus Mais, Reis, Milo, Weizen, aus diesen Getreiden gewonnenen Mehlen, aus diesen Getreiden gewonnenen Stärken, Maltodextrin, Mehlen und Stärken sowie Glukose besteht; und worin die Quelle für assimilierbaren Stickstoff aus der Gruppe ausgewählt ist, die aus Maisquellwasser, hydrolysiertem Kasein, hydrolisierten Sojabohnen, Ammoniumphosphat, Ammoniumsulfat, Ammoniak, Hefeextrakt, einem Proteinhydrolysat und einem Hydrolysat einer Flavobacterium-Biomasse besteht.

31. Futtermittel-Zusammensetzung, umfassend nicht-lebensfähige Zeaxanthin enthaltende Flavobacterium multivorum-Zellen sowie verbleibende Zellreste und unverbrauchte Nährstoffmedium-Feststoffe, die aus der Fermentation von Flavobacterium multivorum-Zellen gewonnen wurden.

32. Zusammensetzung nach Anspruch 31, die eine Geflügel-Futtermittelzusammensetzung, Lachs-Futtermittelzusammensetzung oder Lebensmittelfärbezusammensetzung ist.

33. Futtermittel-Zusammensetzung, die Zeaxanthin enthaltende Flavobacterium multivorum-Zellen und ein Nährstoffmedium umfaßt.

34. Zusammensetzung nach Anspruch 33, worin die Zusammensetzung eine Geflügel-Futtermittelzusammensetzung ist.

35. Verwendung von Flavobacterium multivorum-Zellen bei der Herstellung einer Futtermittel-Zusammensetzung.

## Revendications

1. Procédé pour la production d'une composition contenant de la zéaxanthine comprenant :
la mise en culture d'un micro-organisme de Flavobactérium multivorum, ou ses mutants ou variants, dans un milieu nutritif contenant au moins une source de carbone assimilable et au moins une source d'azote assimilable, où la mise en culture du micro-organisme a pour résultat la production d'une culture comprenant une masse cellulaire contenant de la zéaxanthine et du milieu nutritif résiduel,
et la récupération de la zéaxanthine de la culture.

2. Procédé selon la revendication 1 comprenant de plus la séparation de la masse cellulaire contenant la zéaxanthine du milieu nutritif.

3. Procédé selon la revendication 2 comprenant de plus la séparation de la zéaxanthine de la masse cellulaire.

4. Procédé selon la revendication 1 où la zéaxanthine est récupérée de la culture sous la forme d'une pâte de cellules.

5. Procédé selon l'une quelconque des revendications 1 à 3 où ledit Flavobactérium multivorum, ou son variant ou mutant, a les caractéristiques taxonomiques suivantes : insensible à la pénicilline, insensible à la vancomycine, et insensible à la polymyxine; fortement positif à l'oxydase, positif à la catalase, tiges gram négatif; non-mobile; fortement positif au saccharose; négatif au mannitol; et négatif à l'éthanol; positif à l'urée; et non-protéolytique.

6. Procédé selon l'une quelconque des revendications 1 à 3 où ledit procédé donne au moins 40 g/l de cellules contenant de la zéaxanthine.

7. Procédé selon l'une quelconque des revendications 1 à 3 où ledit milieu nutritif contient de la liqueur de trempage de maïs ou d'autres sources d'azote assimilable et une une source de carbone traitée par une enzyme.

8. Procédé selon la revendication 7 où ladite enzyme est l'a-amylase.

9. Procédé selon l'une quelconque des revendications 1 à 3 où le niveau de glucose dans ledit milieu est inférieur à environ 0,035% en poids.

10. Procédé selon l'une quelconque des revendications 1 à 3 où ladite source de carbone assimilable est sélectionnée dans le groupe consistant en maïs, riz, milo, lait, farines dérivées de ces grains, amidon dérivé de ces grains, malto-dextrine et glucose.

11. Procédé selon l'une quelconque des revendications 1 à 3 où ladite source d'azote assimilable est sélectionnée dans le groupe consistant en liqueur de trempage de maïse, caséine hydrolysée, soja hydrolysé, phosphate d'ammonium, et sulfate d'ammonium, ammoniaque, extrait de levure, un hydrolysat de protéine et un hydrolysat de la biomasse de Flavobactérium.

12. Procédé selon l'une quelconque des revendications 1 à 3 où ladite étape de mise en culture est entreprise à une température comprise entre environ 16°C et 38°C, à un pH compris entre environ 6,0 et environ 8,5 et pendant environ 24 à environ 72 heures.

13. Procédé de la revendication 12 où ledit procédé est sensiblement terminé en environ 32 heures.

14. Procédé selon l'une quelconque des revendications 1 à 3 où ledit milieu nutritif est une solution aqueuse.

15. Procédé selon l'une quelconque des revendications 1 à 3 où ladite mise en culture est entreprise entre environ 22°C et environ 34°C.

16. Procédé selon l'une quelconque des revendications 1 à 3 où ladite mise en culture est entreprise à un pH compris entre environ 6,5 et environ 8,0.

17. Procédé selon l'une quelconque des revendications 1 à 3 où ladite mise en culture est entreprise en environ 24 à environ 72 heures.

18. Procédé selon l'une quelconque des revendications 1 à 3 où ledit procédé est sensiblement terminé en environ 32 heures.

19. Procédé selon l'une quelconque des revendications 1 à 3 consistant de plus à ajouter des enzymes audit milieu nutritif pendant la mise en culture.

20. Procédé de la revendication 19 où ladite enzyme est la glucoamylase, la lipase, ou leurs mélanges.

21. Procédé selon l'une quelconque des revendications 1 à 3 où un promoteur de formation pigmentaire est ajouté audit milieu nutritif.

22. Procédé de la revendication 3 où la zéaxanthine est séparée de ladite masse cellulaire par;
digestion de ladite masse cellulaire avec un solvant organique inerte pour dissoudre la zéaxanthine; et
élimination dudit solvant, de la zéaxanthine.

23. Procédé de la revendication 22, où le solvant est éliminé de la zéaxanthine par évaporation.

24. Procédé selon la revendication 1, où ledit milieu nutritif contient environ 1% en poids à environ 7% en poids d'une source de carbone assimilable et environ 3% en poids à environ 10% en poids d'une source d'azote assimilable.

25. Procédé selon la revendication 1, dans lequel, à la suite de l'étape de mise en culture, la zéaxanthine est extraite au solvant, des cellules de micro-organisme.

26. Procédé de la revendication 1, où il comprend de plus la séparation de ladite masse cellulaire de ladite liqueur de fermentation pour former une pâte de cellules;
la mise en bouillie de ladite pâte;
l'homogénisation de ladite pâte; et
le séchage de ladite pâte.

27. Procédé de la revendication 26 où ladite pâte est mise en bouillie dans des agents protecteurs et un agent émulsionnant.

28. Procédé de la revendication 27 où lesdits agents protecteurs sont des antioxydants, des agents complexants, ou leurs mélanges.

29. Procédé de la revendication 26 où la source de carbone assimilable est traitée par une enzyme.

30. Procédé de la revendication 1, où ladite source de carbone assimilable est sélectionnée dans le groupe consistant en maïs, riz, milo, blé, farines dérivées de ces grains, amidon dérivé de ces grains, malto-dextrine, farines et amidons, et amidons dérivés de ces grains et glucose; et ladite source d'azote assimilable est sélectionnée dans le groupe consistant en liqueur de trempage de maïs, caséine hydrolysée, soja hydrolysé, phosphate d'ammonium, sulfate d'ammonium, extrait de levure, un hydrolysat de protéine et un hydrolysat de la biomasse de Flavobactérium.

31. Composition d'alimentation comprenant des cellules non viables de Flavobactérium multivorum contenant de la zéaxanthine, et des débris cellulaires résiduels et des solides de milieu nutritif non utilisé récupérés de la fermentation des cellules de Flavobactérium multivorum.

32. Composition de la revendication 31 qui est une composition d'aliments pour volaille, une composition d'aliments de salmonidés, ou une composition de coloration des aliments.

33. Composition d'alimentation comprenant des cellules de Flavobactérium multivorum contenant de la zéaxanthine et un milieu nutritif.

34. Composition de la revendication 33 où ladite composition est une composition d'alimentation pour de la volaille.

35. Utilisation des cellules de Flavobactérium multivorum dans la fabrication d'une composition d'alimentation.
